# EUROPEAN PATENT SPECIFICATION

(11) **EP 0 606 924 B1**
(45) Date of publication and mention of the grant of the patent: **04.06.1997**
(21) Application number: 94101361.7
(22) Date of filing: 24.07.1991
(51) Int. Cl.: C07C 249/12

(54) **Process for producing E-isomers of methoxyminoacetamide compounds**
Verfahren zur Herstellung von E-Isomeren von Methoxyiminoacetamidverbindungen
Procédé pour la préparation d'isomères E de méthoxyiminoacétamides

(30) Priority: 26.07.1990 JP 200696/90
(43) Date of publication of application: 20.07.1994
(62) Divisional of application: 91306762.5
(73) Proprietor: SHIONOGI SEIYAKU KABUSHIKI KAISHA trading under the name of SHIONOGI & CO. LTD., Osaka 541 (JP)
(72) Inventor: Takase, Akira, Otsu-shi, Shiga-ken (JP); Nishida, Kuniyoshi, Koga-gun, Shiga-ken (JP); Nagai, Masahiko, Amagasaki-shi, Hyogo-ken (JP); Kai, Hiroyuki, Yamamokoriyama-shi, Nara-ken (JP); Shinomoto, Shoji, Matsubara-shi, Osaku-fu (JP)
(74) Representative: Hardisty, David Robert

(56) References cited:
- EP-A- 0 254 426
- EP-A- 0 398 692
- METHODEN DER ORGANISCHEN CHEMIE (HOUBEN WEYL), 4TH Edition, vol.X/4, 1968, Georg Thieme Verlag, Stuttgart, DE, pages 282 - 294

## Description

The present invention relates to a process for producing methoxyiminoacetamide compounds an E-isomer of the formula: 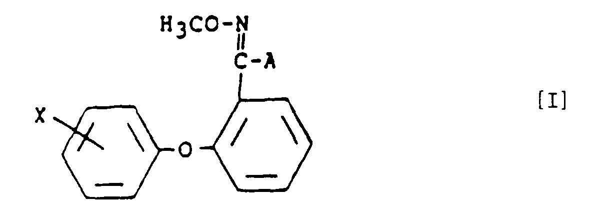 wherein X is hydrogen, C₁ -C₆ alkyl, C₁ -C₆ alkoxy or halogen.

Compounds of the formula: 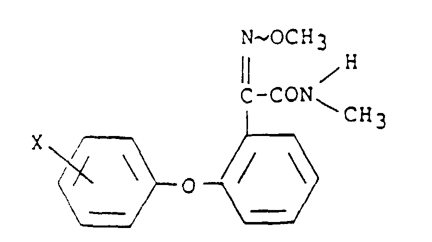 wherein X is hydrogen, C₁ -C₆ alkyl, C₁ -C₆ alkoxy or halogen; and - is any configuration of E-isomer, Z-isomer or a mixture thereof are compounds which have been disclosed for the first time in EP-A-0 398 692 in the name of the present assignee, and they are considered to be remarkably useful compounds as agricultural fungicides having excellent fungicidal activities against microorganisms such as *Piricularia oryzae, Pellucularia sasakii, Pseudoperonospora cubensis* and the like.

In the above application, the compounds of the formula [I] are produced by several routes. However, at this time, the present inventors have found other novel synthetic routes for producing the compounds of the formula [I] which are more economical with minimum by-products.

The present invention provides a novel process for producing certain methoxyiminoacetamide compounds of the formula [I] which are useful for agricultural fungicides.

In other aspects the invention includes a fungicidal composition comprising at least one compound of the formula [I] when produced by the process of the invention together with one or more diluents, excipients or carriers, which composition is useful in a method for controlling phytopathogenic fungi which comprises applying as an active ingredient at least one compound of formula [I] when produced by a process of the invention to a locus where phytopathogenic fungi may propagate or are propagating.

According to the present invention there is provided a process for producing an E-isomer of the formula: 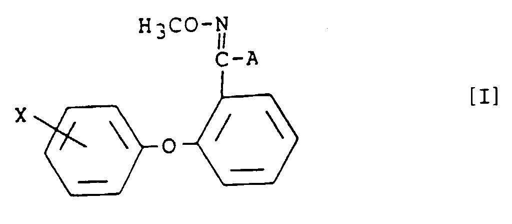 wherein X is hydrogen, C₁ -C₆ alkyl, C₁ -C₆ alkoxy or halogen; A is -CONHCH₃ or -COOR; and R is C₁ -C₆ alkyl, which comprises reacting a Z-isomer of the formula [II]: 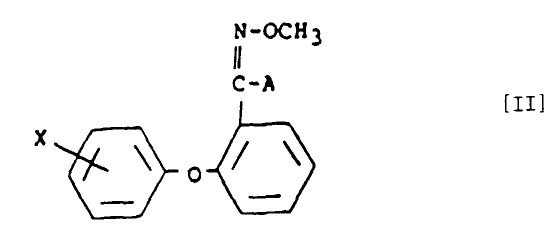 wherein X, A and R are as defined above, with an acid in a C₁ -C₆ alcohol.

The compound of the formula [II] is produced by methylating a compound of the formula [III]: 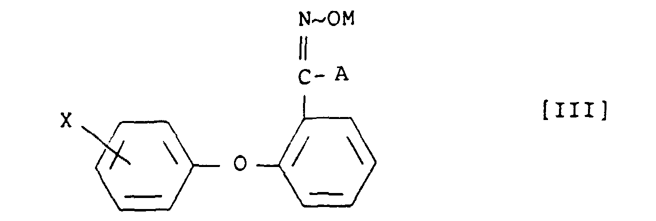 wherein X and A are as defined above and M is hydrogen or an alkali metal, with a compound of the formula:

CH₃-L [IV]

wherein L is halogen or -OSO₂OCH₃.

In each compound disclosed in the present specification, "alkyl" represented by "X" is that having 1 to 6 carbon atoms these being lower alkyl groups, preferably 1 to 4 carbon atoms, for example, methyl, ethyl, propyl, isopropyl, butyl, isobutyl, t-butyl and the like. Examples of alkoxy groups having 1 to 6 carbon atoms, preferably 1 to 4 carbon atoms are, for example, methoxy ethoxy, propoxy, isopropoxy, butoxy, isobutoxy, t-butoxy and the like. Examples of "halogen" include fluorine, chlorine, bromine and iodine. "Alkyl" represented by "R" is that having 1 to 6 carbon atoms as exemplified with respect to "X". Examples of the alkali metal include potassium and sodium. Examples of "halogen" represented by L include the same halogen atoms as those described with respect to the above X.

The compounds of the process thus obtained wherein X is hydrogen can be converted into corresponding compounds wherein X is C₁-C₆ alkyl or halogen by a known method such as alkylation or halogenation. Regarding the compound of the formula [I], the E-isomer (the compounds produced by the process of this invention) and the corresponding Z-isomer can be represented by the following formulas: 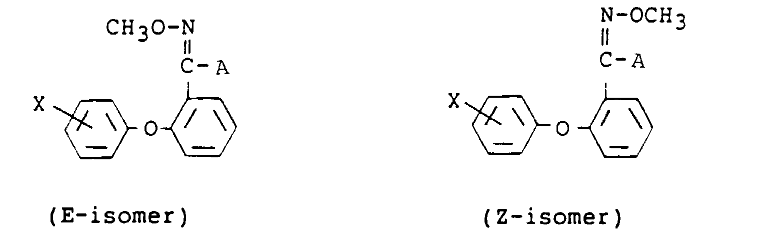 wherein X and A are as defined above.

The Z-isomer of the formula [II}: 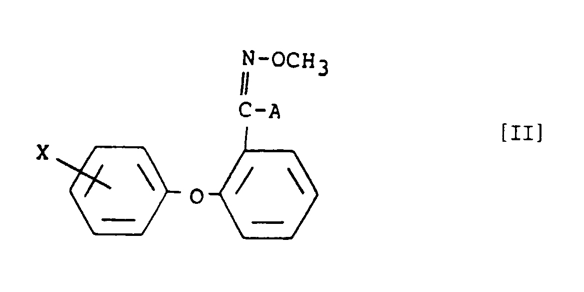 wherein A is -CONHCH₃ or -COOR, can be converted into E-isomer of the Formula (I): 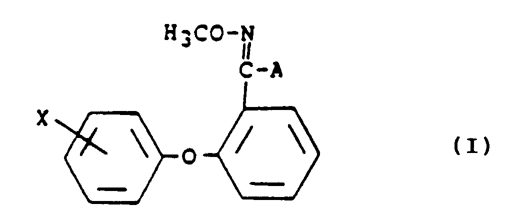 by treating it with an acid in a lower alcohol (a C₁-C₆) alcohol). For example, the treatment can be conducted by reacting the compound of the Formula (II) with 0.1 to 10 moles of an acid such as hydrogen chloride, sulfuric acid or toluenesulfonic acid in a lower alcohol under normal conditions or a sealed tube at 20 to 150°C for is minutes to 48 hours. Further, this reaction can also be applied to a reaction mixture containing both compounds of the formulae [I] and the corresponding Z-isomer as well as crude products.

The compound of the formula [I] thus obtained can be purified, if necessary, according to a conventional method, for example, column chromatography and can be used as an agricultural fungicide according to a known method.

As described above, according to the present invention there is provided a novel process for producing the E-isomer of methoxyiminoacetamide compounds which are useful as agricultural fungicides and the process of the present invention is economical with minimum by-products.

Reference may be made to the parent of this application, EP-B-0 468 775 and to the co-pending divisional application EP-A-0 605 392 for information on procedural chemistry relevant to the present process.

The following Examples further illustrate the present invention in detail but are not to be construed to limit the scope thereof:

### Example 1

### Production of E-α-methoxyimino-2-phenoxyphenylacetic acid methyl ester

To Z-α-methoxyimino-2-phenoxyphenylacetic acid (0.29 g, 1.0 mmole) were added dried methanol (3 ml) and 10% hydrochloric acid solution in methanol (0.36 g, 1.0 mmole) and the mixture was heated under reflux for 6 hours to conduct isomerization.

The reaction mixture was concentrated under reduced pressure, giving a crystalline residue(0.29g). The residue consists of E-isomer and Z-isomer in the ratio of 77.6:22.4, by a ¹H-NMR measurement.

### Example 2

### Production of E-2-methoxyimino-N-methyl-2-(2-phenoxyphenyl)acetamide

To Z-2-methoxyimino-N-methyl-2-(2-phenoxyphenyl)acetamide (0.28 g, 1.0 mmole) were added dried methanol (3 ml) and 10% hydrochloric acid solution in methanol (0.36 g, 1.0 mmole) and the mixture was heated under reflux for 6 hours to conduct isomerization.

The reaction mixture was concentrated under reduced pressure, giving a crystalline residue (0.289 g). The residue consists of E-isomer and Z-isomer in the ratio of 71.5:28.5, by a ¹R-NHR measurement.

## Claims

1. A process for producing a E-isomer of the formula: 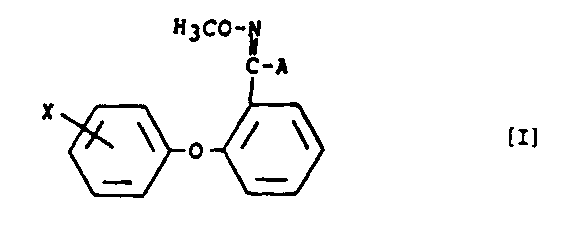 wherein X is hydrogen, C₁-C₆ alkyl, C₁-C₆ alkoxy or halogen; A is -CONHCH₃ or -COOR; and R is C₁-C₆ alkyl, which comprises reacting a Z-isomer of the Formula (II): 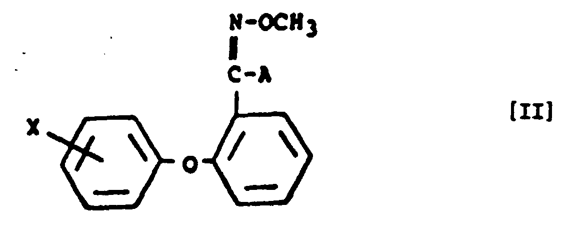 wherein X, A and R are as defined above, with an acid in a C₁-C₆ alcohol.

2. A process according to claim 1, wherein the compound of Formula (II) is reacted with hydrogen chloride, sulfuric acid or toluenesulfonic acid in methanol or ethanol under normal conditions or in a sealed tube at 20 to 150°C for 15 minutes to 48 hours.

3. A process as claimed in claim 2, wherein X is a C₁-C₄ alkyl.

4. A process as claimed in claim 3 wherein X is methyl.

5. A process as claimed in claim 1 or claim 2, wherein X is C₁-C₄ alkoxy.

6. A process as claimed in claim 1 or claim 2, wherein X is hydrogen.

7. A process for making a fungicidal composition wherein a compound of formula [I] is prepared by a process as claimed in any one of the preceding claims and thereafter the resulting compound is formulated into a fungicidal composition with one or more diluents, excipients or carriers.

## Patentansprüche

1. Verfahren zur Herstellung eines E-Isomers der Formel: 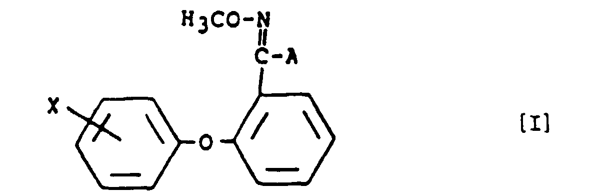 in der X ein Wasserstoffatom, einen C₁-C₆-Alkylrest, einen C₁-C₆-Alkoxyrest oder ein Halogenatom bedeutet, A eine Gruppe -CONHCH₃ oder einen Rest -COOR darstellt und R einen C₁-C₆-Alkylrest bedeutet, das die Umsetzung eines Z-Isomers der Formel [VI]: 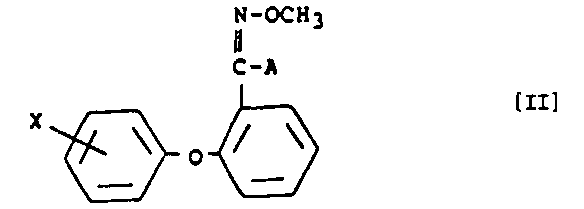 in der X, A und R wie vorstehend definiert sind, mit einer Säure in einem C₁-C₆-Alkohol umfaßt.

2. Verfahren nach Anspruch 1, wobei die Verbindung der Formel [VI] mit Salzsäure, Schwefelsäure oder Toluolsulfonsäure in Methanol oder Ethanol unter normalen Bedingungen oder in einem Bombenrohr bei 20 bis 150°C 15 Minuten bis 48 Stunden umgesetzt wird.

3. Verfahren nach Anspruch 2, wobei X einen C₁-C₄-Alkylrest bedeutet.

4. Verfahren nach Anspruch 3, wobei X eine Methylgruppe darstellt.

5. Verfahren nach Anspruch 1 oder Anspruch 2, wobei X einen C₁-C₄-Alkoxyrest bedeutet.

6. Verfahren nach Anspruch 1 oder Anspruch 2, wobei X ein Wasserstoffatom darstellt.

7. Verfahren zum Herstellen einer fungiziden Zusammensetzung, wobei eine Verbindung der Formel (I) durch ein Verfahren nach einem der vorhergehenden Ansprüche hergestellt wird und die erhaltene Verbindung danach mit einem oder mehreren Verdünnungsmitteln, Vehikeln oder Trägern in eine fungizide Zusammensetzung formuliert wird.

## Revendications

1. Procédé de production d'un isomère E de formule : 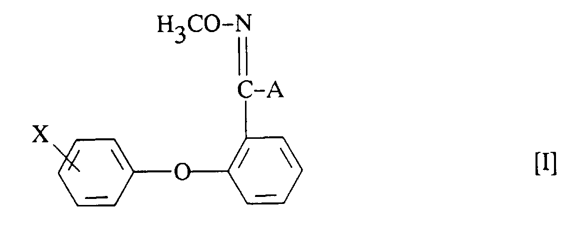 où X est un hydrogène, un alkyle C₁-C₆, un alcoxy C₁-C₆ ou un halogène ; A est -CONHCH₃ ou -COOR; et R est un alkyle C₁-C₆, qui comprend de faire réagir un isomère Z de formule [II]: 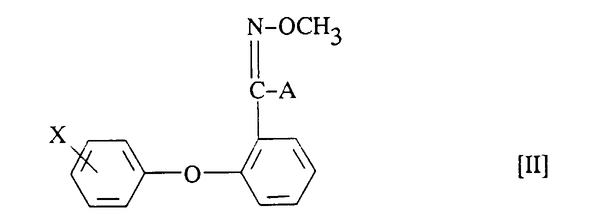 où X, A et R sont tels que définis ci-dessus, avec un acide dans un alcool C₁-C₆.

2. Procédé selon la revendication 1, où le composé de formule [II] est mis à réagir avec du chlorure d'hydrogène, de l'acide sulfurique ou de l'acide toluènesulfonique dans le méthanol ou l'éthanol dans des conditions ambiantes ou dans un tube scellé entre 20 et 150°C pendant 15 min à 48 h.

3. Procédé selon la revendication 2, où X est un alkyle C₁-C₄.

4. Procédé selon la revendication 3, où X est un méthyle.

5. Procédé selon la revendication 1 ou la revendication 2, où X est un alcoxy C₁-C₄.

6. Procédé selon la revendication 1 ou la revendication 2, où X est un hydrogène.

7. Procédé de production d'une composition fongicide où on prépare un composé de formule [I] selon un procédé revendiqué dans l'une quelconque des revendications précédentes et ensuite on formule le composé obtenu dans une composition fongicide avec un ou plusieurs diluants, excipients ou véhicules.
